# EUROPÄISCHE PATENTANMELDUNG

(11) **EP 1 770 427 A2**
(43) Veröffentlichungstag der Anmeldung: **04.04.2007**
(21) Anmeldenummer: 06026761.4
(22) Anmeldetag: 24.09.2001
(51) Int. Cl.: G02B 21/22, G02B 21/00, A61B 3/13

(54) **Stereo-Mikroskopieanordnung**

(30) Priorität: 26.09.2000 DE 10047617
(62) Teilanmeldung aus: 01122891.3
(71) Anmelder: Carl Zeiss Surgical GmbH, 73447 Oberkochen (DE)
(72) Erfinder: Strähle, Fritz, Dr., 73540 Heubach (DE)
(74) Vertreter: Schorr, Frank Jürgen

(57) **Zusammenfassung**

Es wird eine Stereo-Mikroskopieanordnung zur Betrachtung eines Objekts oder eines von einem Objekt erzeugten Zwischenbilds vorgeschlagen, umfassend eine Objektivanordnung mit einer Objektebene zur Anordnung des zu betrachtenden Objekts bzw. Zwischenbilds, eine linke Okularanordnung, der ein in die Objektivanordnung links eintretendes Strahlenbündel zugeführt wird, und eine rechte Okularanordnung, der ein in die Objektivanordnung rechts eintretendes Strahlenbündel zugeführt wird, wobei in den Strahlengängen der Okularanordnungen ein Strahlvertauschungssystem (1) vorgesehen ist, welches das der linken Okularanordnung zugeführte Strahlenbündel (5) in die rechte Okularanordnung spiegelt und welches das der rechten Okularanordnung zugeführte Strahlenbündel in die linke Okularanordnung spiegelt, wobei die dem Strahlvertauschungssystem zugeführten Strahlenbündel und aus dem Strahlvertauschungssystem austretende Strahlenbündel einander gleiche Bildorientierungen aufweisen, wobei das Strahlvertauschungssystem solche optischen Komponenten ((15,41), (35,61)) aufweist, die bezüglich einer Mittelebene (3) zwischen den Strahlenbündeln auf diese symmetrisch wirken, sowie auch solche optischen Komponenten (25, 51) aufweist, die bezüglich der Mittelebene (3) auf die Strahlenbündel unsymmetrisch wirken.

## Beschreibung

Die vorliegende Erfindung betrifft eine Stereo-Mikroskopieanordnung zur Betrachtung eines Objekts oder eines von einem Objekt erzeugten Zwischenbilds. Hierbei kann das Zwischenbild insbesondere ein höhen- und seitenverkehrtes Abbild eines Objekts sein, und die Mikroskopieanordnung kann zum Einsatz in der Mikrochirurgie, insbesondere der Augenchirurgie, vorgesehen sein.

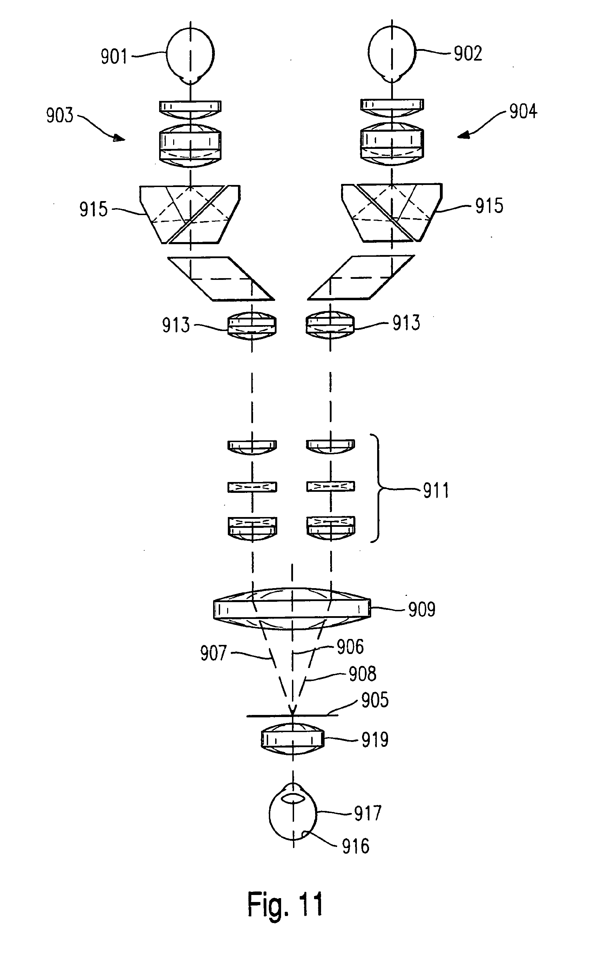

In Figur 11 ist ein Strahlengang eines herkömmlichen Stereo-Mikroskops schematisch dargestellt. Bei dem herkömmlichen Stereo-Mikroskop blickt der Betrachter mit seinen beiden Augen 901 und 902 in ein linkes Okular 903 bzw. ein rechtes Okular 904 und betrachtet dabei beispielsweise ein Objekt, das in einer Objektebene 905 des Mikroskops angeordnet ist. Die Okulare 903 und 904, Bildumkehrprismen 915 und die Tubuslinsen 913 bilden zusammen den Tubus, der funktionell ein binokulares Kepler-Fernrohr darstellt. Der Betrachter nimmt das Objekt mit einem stereoskopischen Raumeindruck wahr, da ein von dem Objekt ausgehendes Strahlenbündel 907, welches bezüglich einer Mittelebene 906 des Mikroskops nach links zu einer Sammellinse 909 des Mikroskops verläuft, links in diese Sammellinse 909 eintritt und in das linke Okular 903 abgebildet wird, während ein von dem Objekt bezüglich der Mittelebene 906 nach rechts ausgehendes Strahlenbündel 908 auch rechts in die Sammellinse 909 eintritt und in das rechte Okular 904 abgebildet wird. Die Objektivanordnung umfaßt zur Änderung der Vergrößerung ferner ein afokales Zoomsystem 911, und die Okulare 903, 904 umfassen jeweils eine Tubuslinse 913 und ein Schmidt-Pechan-Prisma 915. Die Schmidt-Pechan-Prismen sind notwendig, um das Bild des Objekts, dessen Bildorientierung durch das Objektiv umgekehrt wurde, wieder höhen- und seitenrichtig zu stellen.

Das herkömmliche Stereo-Mikroskop kann auch in der Augenchirurgie zur Betrachtung eines Augenfundus 916 eines Patientenauges 917 eingesetzt werden, wozu vor dem Patientenauge eine Ophthalmoskopierlinse 919 angeordnet wird, welche in der Objektebene 905 des Mikroskops ein Zwischenbild des Augenfundus 916 erzeugt. Das Zwischenbild des Augenfundus wird dann vom Operateur durch das Stereo-Mikroskop betrachtet.

Bei der in Figur 11 gezeigten Anordnung des Stereo-Mikroskops und der Ophthalmoskopierlinse stellt sich auf Grund der Änderungen der Bildorientierung und stereoskopischen Blickrichtung durch die Ophthalmoskopierlinse der Augenfundus für den Operateur höhen- und seitenverkehrt und zudem pseudo-stereoskopisch dar, d.h. in der Tiefenwahrnehmung ist für den Operateur vorne und hinten vertauscht. Um dieses Problem zu beheben, muss zum einen die richtige Bildorientierung wieder hergestellt werden und zum anderen müssen die beiden Stereokanäle des Mikroskops vertauscht werden, d.h. das von der Objektebene 905 nach links ausgehende Strahlenbündel 907 muss dem rechten Auge 902 des Operateurs zugeführt werden, und das nach rechts ausgehende Strahlenbündel 908 muss dessen linkem Auge 901 zugeführt werden.

Hierzu sind aus dem Stand der Technik eine Reihe von Möglichkeiten bekannt:
Gemäß DE 41 14 646 A1 kann zwischen Ophthalmoskopierlinse und Sammellinse des Objekts ein Schmidt-Pechan-Prisma mit Dachkante angeordnet werden.

Gemäß DE 38 26 069 A1 kann in dem parallelen Strahlengang zwischen Vergrößerungswechsler und Tubuslinsen der Okulare ein Spiegelsystem angeordnet werden, welches die Bildorientierung ändert und den rechten mit dem linken Strahlengang vertauscht.

Nachteile bei den bekannten Lösungen sind zum einen eine Behinderung des Zugangs zum Operationsfeld, wenn die Bildvertauschung nahe der Ophthalmoskopierlinse vorgenommen wird, und zum anderen eine ergonomisch ungünstige Vergrößerung der Bauhöhe des Mikroskops, wenn das Spiegelsystem zwischen afokalem Zoomsystem und Tubus angebracht ist.

Es ist eine Aufgabe der vorliegenden Erfindung, eine verbesserte Stereo-Mikroskopieanordnung zur Betrachtung eines Objekts oder eines von einem Objekt erzeugten Zwischenbilds bereitzustellen, welches sich insbesondere zur Betrachtung eines Zwischenbilds mit verkehrter Bildorientierung und Pseudostereoskopie eignet.

Gemäß einem ersten Aspekt geht die Erfindung aus von einer Stereo-Mikroskopanordnung mit einer Objektivanordnung und einer Objektebene zur Anordnung des zu betrachtenden Objekts bzw. Zwischenbilds, einer linken Okularanordnung, der ein in die Objektivanordnung links eintretendes Strahlenbündel zugeführt wird, und einer rechten Okularanordnung, der ein in die Objektivanordnung rechts eintretendes Strahlenbündel zugeführt wird.

Die Erfindung zeichnet sich dabei dadurch aus, daß ein in Strahlengängen der Okularanordnungen angeordnetes Strahlvertauschungssystem vorgesehen ist, um die Stereokanäle zu vertauschen, d.h. um das der linken Okularanordnung zugeführte Strahlenbündel in die rechte Okularanordnung überzuführen und um das der rechten Okularanordnung zugeführte Strahlenbündel der linken Okularanordnung zuzuführen. Hierbei ändert allerdings das Strahlvertauschungssystem die Bildorientierung nicht, d.h. in das Strahlvertauschungssystem eintretende Strahlenbündel und aus diesem austretende Strahlenbündel haben einander gleiche Bildorientierungen. Es ist in der Okularanordnung auch kein weiteres System vorgesehen, um die Bildorientierung zu ändern.

Der Erfindung liegt der Gedanke zugrunde, daß bei dem herkömmlichen Stereo-Mikroskop, wie eingehend bereits erläutert, innerhalb der Okulare bzw. des Tubus eine Vorrichtung zur Bildumkehr vorgesehen ist. Ist nun aber diese Bildumkehr bei der Betrachtung des Zwischenbildes durch die Ophthalmoskopierlinse bereits vollzogen, so muss im Tubus keine Änderung der Bildorientierung mehr vorgenommen werden. Zur Behebung des pseudo-stereoskopischen Raumeffekts ist dann anstatt der Bildumkehr lediglich die Vertauschung der beiden Stereokanäle notwendig.

Bevorzugterweise ist das Strahlvertauschungssystem aus den Strahlengängen der Okularanordnungen entfernbar und ersetzbar durch ein Bildumkehrsystem, das die Bildorientierungen der den Okularanordnungen jeweils zugeführten Strahlenbündel umkehrt, ohne eine Vertauschung der Stereokanäle vorzunehmen. Damit ist die Stereo-Mikroskopieanordnung von einem ersten Beobachtungsmodus zur Betrachtung eines höhen- und seitenverkehrten Zwischenbilds, bei dem das Strahlvertauschungssystem in die Strahlengänge der Okularanordnungen eingeführt ist, in einen zweiten Beobachtungsmodus zur Betrachtung eines Objekts in der Objektebene überführbar, bei der das Bildumkehrsystem in die Strahlengänge eingeführt ist.

Hierbei ist es vorteilhaft, wenn optisch wirksame Medien und Geometrien des Strahlvertauschungssystems und des Bildumkehrsystems derart aufeinander abgestimmt sind, daß Strahlenbündel, welche die Systeme durchsetzen, in diesen im wesentlichen auf die gleichen Okularzwischenbildlagen fokussiert sind. Hierdurch wird erreicht, daß bei einem Wechsel von dem einen Beobachtungsmodus in den anderen im wesentlichen keine Nachfokussierungen an der Stereo-Mikroskopieanordnung notwendig sind, um für beide Augen des Betrachters jeweils ein scharfes Bild zu erhalten.

Hierdurch ist es insbesondere auch möglich, das Strahlvertauschungssystem oder/und das Bildumkehrsystem platzsparend in einem konvergenten oder einem divergenten Strahlengang der Okularanordnungen vorzusehen, welche insbesondere als binokulare Kepler-Fernrohre ausgebildet sein können.

Vorteilhaft ist es weiterhin, eine Justierung der Okularzwischenbildlagen dadurch zu ermöglichen, daß an dem Strahlvertauschungssystem eine Einstellvorrichtung vorgesehen ist, um einen Unterschied, den das Strahlvertauschungssystem für die diesem zugeführten Strahlenbündel hinsichtlich deren in dem Strahlvertauschungssystem zurückgelegten optischen Weglängen bereitstellt, auf einen gewünschten Wert einzustellen. Dieser gewünschte Wert ist insbesondere im wesentlichen null, d.h. beide Strahlenbündel legen durch das Strahlvertauschungssystem eine im wesentlichen gleiche optische Weglänge zurück, weichen jedoch infolge unvermeidbarer Fertigungstoleranzen der optischen Komponenten geringfügig voneinander ab.

Erfindungsgemäß umfaßt das Strahlvertauschungssystem für die beiden diesem zugeführten Strahlenbündel, nämlich das linke Strahlenbündel und das rechte Strahlenbündel, optisch wirksame Komponenten, die bezüglich einer Mittelebene zwischen den beiden Strahlenbündeln auf diese symmetrisch wirken sowie auch Komponenten, die auf die Strahlenbündel unsymmetrisch wirken. Bei einem besonders bevorzugten Strahlenvertauschungssystem ist hierbei vorgesehen, daß das linke Strahlenbündel durch einen Spiegel zunächst nach rechts abgelenkt wird, während das rechte Strahlenbündel durch einen weiteren Spiegel zunächst nach links abgelenkt wird. Diese beiden Spiegel wirken also bezüglich der Mittelebene auf die beiden Strahlenbündel symmetrisch. Sodann wird das linke Strahlenbündel durch zwei aufeinanderfolgende Spiegel zweimal nach links abgelenkt, während das rechte Strahlenbündel durch zwei aufeinanderfolgende Spiegel ebenfalls zweimal nach links abgelenkt wird. Diese beiden Spiegelpaare wirken somit bezüglich der Mittelebene auf die beiden Strahlenbündel unsymmetrisch. Daraufhin wird das linke Strahlenbündel durch noch einen weiteren Spiegel wieder nach rechts abgelenkt, während das rechte Strahlenbündel durch noch einen weiteren Spiegel wieder nach links abgelenkt wird, bevor die beiden Strahlenbündel das Strahlvertauschungssystem verlassen. Die beiden letztgenannten Spiegel wirken auf die beiden Strahlenbündel wiederum symmetrisch.

Gemäß einem zweiten Aspekt geht die Erfindung aus von einer Stereo-Mikroskopieanordnung mit einer Objektivanordnung und einer Objektebene zur Anordnung eines zu betrachtenden Objekts bzw. Zwischenbilds, wobei ein Strahlvertauschungs- und Bildumkehrsystem zwischen Objektebene und Eintrittslinse der Objektivanordnung angeordnet ist. Das Strahlvertauschungs- und Bildumkehrsystem führt ein bezüglich einer Mittelebene nach links in Richtung zu der Objektivanordnung gerichtetes Strahlenbündel der Objektivanordnung rechts zu, es führt ein bezüglich der Mittelebene nach rechts in Richtung zu der Objektivanordnung gerichtetes Strahlenbündel der Objektivanordnung links zu, und es kehrt die Bildorientierungen der beiden Strahlenbündel jeweils um. Damit ist die Stereo-Mikroskopieanordnung besonders zur Betrachtung eines höhen- und seitenverkehrten Zwischenbilds geeignet.

Beispielhaft ist hierbei vorgesehen, daß das Strahlvertauschungs- und Bildumkehrsystem wenigstens ein Porro-Prisma zweiter Art umfaßt.

Es hat sich herausgestellt, daß das Porro-Prisma zweiter Art eine besonders wirksame optische Komponente ist, um einen für die Strahlvertauschung notwendigen Versatz zwischen eintretendem und austretendem Strahlenbündel bereitzustellen und gleichzeitig die gewünschte Bildumkehr zu erzielen. Das Porro-Prisma zweiter Art ist vergleichsweise kompakt mit einer vergleichsweise kurzen optischen Weglänge, so daß eine Behinderung des freien Arbeitsraumes und auch sichtbare Störungen des betrachteten Bildes, wie etwa Bildfeldbeschnitt, weitgehend vermeidbar sind.

Vorzugsweise ist hierbei für einen jeden der beiden Strahlenbündel ein diesem separat zugeordnetes Porro-Prisma zweiter Art vorgesehen, wodurch die Bildumkehr und Strahlvertauschung mit optischen Komponenten erreicht wird, die zum gesamten Lichtweg der Strahlenbündel mit nur einer vergleichsweise kurzen optischen Weglänge beitragen.

Eine hierzu alternative Anordnung ist dann vorteilhaft, wenn die Stereo-Mikroskopieanordnung zur Benutzung durch zwei Beobachter ausgelegt ist. Der Objektivanordnung bezüglich einer Mittelebene links und rechts zugeführte Strahlenbündel werden dabei zu dem linken bzw. rechten Auge eines ersten Beobachters abgebildet, während der Objektivanordnung zentral zur Mittelebene oben und unten zugeführte Strahlenbündel auf das linke bzw. rechte Auge des zweiten Beobachters abgebildet werden.

Hierbei ist vorzugsweise ein erstes Porro-Prisma zweiter Art vorgesehen, um die links und rechts zugeführten Strahlenbündel für den ersten Beobachter sowie das unten zugeführte Strahlenbündel für den zweiten Beobachter abzubilden, während ein zweites Porro-Prisma zweiter Art vorgesehen ist, um das oben zugeführte Strahlenbündel für den zweiten Beobachter abzubilden. Hierdurch ist eine einfache Anordnung geschaffen, die für zwei unabhängige Beobachter die Strahlvertauschung und Bildumkehr auf einfache Weise bewerkstelligt.

Gemäß einem dritten Aspekt geht die Erfindung aus von einer Stereo-Mikroskopieanordnung zur abwechselnden Betrachtung eines in einer Objektebene angeordneten ersten Objekts, beispielsweise der Cornea eines Auges, und eines in die Objektebene abgebildeten Zwischenbilds eines zweiten Objekts, beispielsweise eines Augenfundus des Auges. Zur Betrachtung des Zwischenbilds des zweiten Objekts werden in den Strahlengang vor dem Objektiv zum einen ein Umkehr-Linsensystem, beispielsweise Ophthalmoskopievorsatz, zur Erzeugung des höhen- und seitenverkehrten Zwischenbilds des zweiten Objekts und ein Strahlvertauschungs- und Bildumkehrsystem zur Bildumkehr und Pupillenvertauschung des Zwischenbilds eingebracht. Zur Betrachtung des ersten Objekts werden die beiden Komponenten Strahlvertauschungs- und Bildumkehrsystem und Umkehr-Linsensystem aus dem Strahlengang zwischen Auge und Objektiv entfernt.

Beispielhaft ist hierbei vorgesehen, daß der Abstand zwischen dem Objektiv und den Objekten bei der Überführung von dem einen Betrachtungsmodus in den anderen im wesentlichen nicht geändert werden muss, um scharfe Bilder zum einen von dem ersten Objekt, beispielsweise dem Bereich der Cornea, und zum anderen von dem zweiten Objekt, beispielsweise dem Bereich des Augenfundus, zu erhalten. Dies wird z.B. dadurch erreicht, daß die geometrischen Abmessungen und optischen Medien der Strahlvertauschungs- und Bildumkehranordnung und die Brennweiten des Umkehr-Linsensystems entsprechend aufeinander abgestimmt sind.

Wesentlich ist hierbei, daß vor allem die optische Weglänge des Strahlvertauschungs- und Bildumkehrsystems beispielsweise durch gezielte Auswahl der hierfür verwendeten optischen Medien derart gestaltet ist, daß die Objektebene von dem Objektiv bei in den Strahlengang eingefügten Strahlvertauschungs- und Bildumkehrsystem einen Abstand aufweist, der sich von dem Abstand der Objektebene von dem Objektiv bei aus dem Strahlengang entferntem Strahlvertauschungs- und Bildumkehrsystem um einen Wert unterscheidet, der durch die beabsichtigten Beobachtungspositionen und dabei insbesondere durch Abstände zwischen den zu beobachtenden Objekten und/oder zu deren Beobachtung verwendeter Hilfsmittel bestimmt ist. Im Fall der Augenchirurgie sind die hierfür relevanten Abstände zwischen den zu beobachtenden Objekten im wesentlichen der Abstand zwischen Cornea und dem Zwischenbild des Augenfundus, und die bei der Beobachtung des Objekts verwendeten Hilfsmittel sind im wesentlichen der Ophthalmoskopievorsatz.

Der Abstand zwischen Objektiv und Objektebene bei in den Strahlengang eingefügtem Strahlvertauschungs- und Bildumkehrsystem weist somit eine Differenz zu dem Abstand zwischen Objektiv und Objektebene bei aus dem Strahlengang entferntem Strahlvertauschungs- und Bildumkehrsystem auf. Vorteilhafterweise liegt diese Differenz zwischen den beiden Abständen in einem Bereich von 15 mm bis 40 mm, vorzugweise 20 mm bis 30 mm und weiter bevorzugt 24 mm bis 26 mm.

Gemäß einem vierten Aspekt geht die Erfindung aus von einer Stereo-Mikroskopieanordnung mit einer Objektivanordnung und einer Objektebene zur Anordnung des zu betrachtenden Objekts bzw. Zwischenbilds und einer linken und einer rechten Okularanordnung, sowie einem Strahlvertauschungs- und Bildumkehrsystem, welches ein in die Objektivanordnung bezüglich einer Mittelebene links eintretendes Strahlenbündel der rechten Okularanordnung zuführt und welches ein in die Objektivanordnung entsprechend rechts eintretendes Strahlenbündel der linken Okularanordnung zuführt und dabei die Bildorientierungen der beiden Strahlenbündel jeweils umkehrt.

Beispielhaft ist hierbei vorgesehen, daß das Strahlvertauschungs- und Bildumkehrsystem ein Paar verkürzter Porro-Prismen zweiter Art umfaßt. Wie bereits vorangehend schon erläutert, ist das Porro-Prisma zweiter Art besonders dafür geeignet, den für die Strahlvertauschung notwendigen Strahlversatz bei gleichzeitiger Bildumkehr bereitzustellen. Erfindungsgemäß werden weitere Verbesserungen der Bildqualität hierbei dadurch erzielt, daß das herkömmliche Porro-Prisma zweiter Art ersetzt wird durch ein sogenanntes "verkürztes" Porro-Prisma zweiter Art. Dieses verkürzte Porro-Prisma zweiter Art stellt bei gleicher optischer Aufgabe, also Strahlversatz und Bildumkehr, im Vergleich zu dem normalen Porro-Prisma zweiter Art eine verkürzte optische Weglänge bereit, so daß eine Reihe von Beschränkungen durch Bauhöhe und sichtbare Bildstörungen bei der erfindungsgemäßen Ausgestaltung der Stereo-Mikroskopieanordnung vermindert sind.

Gemäß einem fünften Aspekt geht die Erfindung aus von einer Stereo-Mikroskopieanordnung mit einer Objektivanordnung und einer Objektebene zur Anordnung des zu betrachtenden Objekts bzw. Zwischenbilds, einer linken und rechten Okularanordnung und einem im Strahlengang zwischen der Objektivanordnung und der Okularanordnung angeordnetem Vergrößerungsänderungssystem sowie einem Strahlvertauschungs- und Bildumkehrsystem.

Beispielhaft ist hierbei vorgesehen, daß das Strahlvertauschungs- und Bildumkehrsystem im Strahlengang vor dem Vergrößerungsänderungssystem und nach der Objektivanordnung vorgesehen ist. Hierdurch lassen sich durch das Strahlvertauschungs- und Bildumkehrsystem hervorgerufene Bildstörungen, wie etwa Vignettierung, vorteilhaft reduzieren.

Im Folgenden werden Ausführungsbeispiele der Erfindung anhand von Zeichnungen näher erläutert. Hierbei zeigt:
- Figur 1: ein Strahlvertauschungssystem zur Verwendung in einer Stereo-Mikroskopieanordnung gemäß einer ersten Ausführungsform der Erfindung,
- Figur 2a: eine Teildarstellung des Strahlvertauschungssystems aus Figur 1 in Anordnung in einem konvergenten Strahlengang,
- Figur 2b: den konvergenten Strahlengang aus Figur 2a bei aus dem Strahlengang entferntem Strahlvertauschungssystem und einem dafür eingefügten Bildumkehrsystem,
- Figur 3: eine Teilansicht eines Strahlengangs einer Stereo-Mikroskopieanordnung gemäß einer zweiten Ausführungsform der Erfindung,
- Figur 4: eine schematische Darstellung eines Ophthalmoskopievorsatzes zur Verwendung mit der erfindungsgemäßen Stereo-Mikroskopieanordnung,
- Figur 5: eine Darstellung eines Strahlengangs durch ein Porro-Prisma zweiter Art,
- Figur 6a: eine Darstellung des Strahlengangs zwischen Objektebene und Objektiv bei einem aus dem Strahlengang entfernten Strahlvertauschungs- und Bildumkehrsystem bei der Ausführungsform gemäß Figur 3,
- Figur 6b: eine der Figur 6a entsprechende Darstellung mit in den Strahlengang eingesetztem Strahlvertauschungs- und Bildumkehrsystem,
- Figur 6c: eine der Figur 6b entsprechende Darstellung, wobei der Strahlengang durch das Strahlvertauschungs- und Bildumkehrsystem entfaltet dargestellt ist,
- Figur 7: eine Detailansicht eines Strahlengangs durch eine Stereo-Mikroskopieanordnung gemäß einer vierten Ausführungsform der Erfindung,
- Figur 8: eine schematische Darstellung der Anordnung von Porro-Prismen zweiter Art vor dem Objektiv der in Figur 7 dargestellten Stereo-Mikroskopieanordnung,
- Figur 9: eine räumliche Teildarstellung der in Figur 7 gezeigten Ausführungsform,
- Figur 10: ein verkürztes Porro-Prisma zweiter Art zur Verwendung bei einer weiteren Ausführungsform der erfindungsgemäßen Stereo-Mikroskopieanordnung,
- Figur 11: eine Stereo-Mikroskopieanordnung gemäß dem Stand der Technik und
- Figur 12: eine Seitenansicht einer Stereo-Mikroskopieanordnung gemäß einer weiteren Ausführungsform der Erfindung.

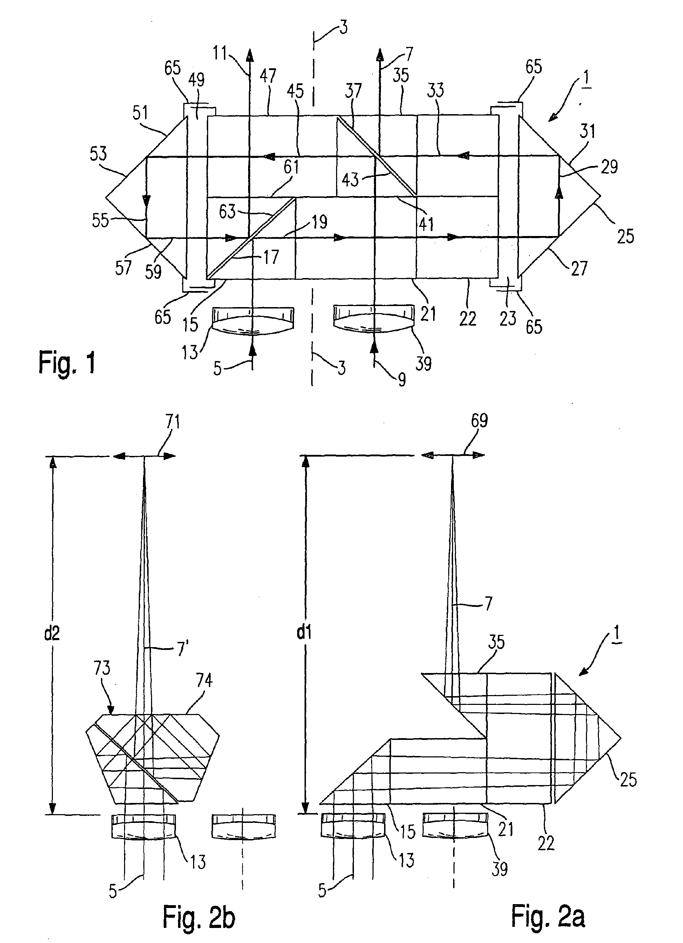

Als eine erste Ausführungsform der Erfindung ist nachfolgend unter Bezugnahme auf die Figuren 1 und 2 ein Stereo-Mikroskop beschrieben, welches wahlweise in zwei Beobachtungsmodi einsetzbar ist. In einem ersten Beobachtungsmodus dient das Stereo-Mikroskop der Betrachtung eines höhen- und seitenverkehrten Zwischenbilds, wie es beispielsweise durch einen Ophthalmoskopievorsatz zur Abbildung eines Augenfundus in einer Objektebene des Mikroskops erzeugt wird, und in einem zweiten Beobachtungsmodus dient das Mikroskop der direkten Betrachtung eines in der Objektebene angeordneten Objekts.

Der Ophthalmoskopievorsatz erzeugt für einen Beobachter ein höhen- und seitenverkehrtes Abbild des Augenfundus als Zwischenbild in pseudo-stereoskopischer räumlicher Darstellung. Um diesen störenden Effekt zu vermeiden, ist in den Strahlengang des Stereo-Mikroskops ein Strahlvertauschungssystem 1 gemäß Figur 1 eingefügt. Das Strahlvertauschungssystem überführt ein bezüglich einer Mittelebene 3 links in das Strahlvertauschungssystem eintretendes Strahlenbündel 5 in ein rechts aus diesem austretendes Strahlenbündel 7, und es überführt ein bezüglich der Mittelebene 3 rechts in das Strahlvertauschungssystem 1 eintretendes Strahlenbündel 9 in ein bezüglich der Mittelebene 3 links aus diesem austretendes Strahlenbündel 11. Hierbei weisen die eintretenden Strahlenbündel 5, 9 und die austretenden Strahlenbündel 7, 11 einander gleiche Bildorientierungen auf, d.h. eine Bildumkehr findet durch das Strahlvertauschungssystem 1 nicht statt.

Das Strahlvertauschungssystem 1 ist aus Glasklötzen und Glasprismen aufgebaut, an deren Innenflächen die Strahlenbündel reflektiert werden.

Der Strahlenverlauf in dem Strahlvertauschungssystem 1 ist in Figur 1 anhand des Verlaufs von Zentralstrahlen der Strahlenbündel 5 und 9 dargestellt.

Nach Durchlaufen einer Tubuslinse 13 einer linken Okularanordnung des Stereo-Mikroskops tritt der Strahl 5 in ein 90° Prisma 15 ein. Das 90° Prisma 15 weist ein unter 45° zur Richtung des Strahls 5 derart angeordnete Spiegelfläche 17 auf, daß der Strahl 5 um 90° nach rechts in einen Strahl 19 abgelenkt wird. Der Strahl 19 verläßt das 90°-Prisma 15 und durchsetzt nacheinander zwei Glasblöcke 21 und 22, wobei das 90°-Prisma 15 und die Glasblöcke 21, 22 jeweils verkittet sind. Nach Verlassen des Glasblocks 22 durchsetzt der Strahl 19 einen Luftspalt 23 und tritt daraufhin in ein 180°-Prisma 25 ein. An einer ersten Spiegelfläche 27 des 180°-Prismas 25 wird der Strahl 19 um 90° nach links in einen Strahl 29 reflektiert, der sich somit parallel zu der ursprünglichen Richtung des Strahls 5 ausbreitet und der dann eine zweite Spiegelfläche 31 des 180°-Prismas 25 trifft, welche ihn um weitere 90° nach links in einen Strahl 33 reflektiert, der sich antiparallel zu der Richtung des Strahls 19 ausbreitet. Der Strahl 33 verläßt das 180°-Prisma 25 und durchsetzt zunächst den Luftspalt 23 und sodann den Glasblock 22, woraufhin er in ein 90°-Prisma 35 eintritt, welches mit dem Glasblock 22 verkittet ist. An einer Spiegelfläche 37 des 90°-Prismas 35 wird der Strahl 33 um 90° nach rechts reflektiert und verläßt das 90°-Prisma 35 und damit das Strahlvertauschungssystem 1 als Strahl 7 in der rechten Okularanordnung.

Der Zentralstrahl des rechten Strahlenbündels tritt nach Durchlaufen einer Tubuslinse 39 der rechten Okularanordnung von unten nach oben kommend in den Glasblock 21 ein, durchsetzt diesen und tritt dann in ein 90°-Prisma 41 mit einer Spiegelfläche 43 ein, an welcher der Strahl 9 um 90° nach links in einen Strahl 45 reflektiert wird. Hierbei ist das 90°-Prisma 41 mit dem Glasblock 21 verkittet, und ferner sind die 90°-Prismen 41 und 35 derart aneinandergefügt, daß deren Spiegelflächen 43 und 37 mit lediglich einem geringen Abstand voneinander angeordnet sind. Nach Verlassen des 90°-Prismas 41 durchsetzt der Strahl 45 ein mit dem 90°-Prisma 41 verkitteten Glasblock 47, woraufhin der Strahl 45 einen Luftspalt 49 durchsetzt und in ein 180°-Prisma 51 eintritt. An einer Spiegelfläche 53 des 180°-Prismas 51 wird der Strahl 45 um 90° nach links in einen Strahl 55 reflektiert, der sich somit antiparallel zu der Richtung des Strahls 9 ausbreitet und sodann von einer zweiten Spiegelfläche 57 des 180°-Prismas 53 um weitere 90° nach links in einen Strahl 59 reflektiert wird, so daß er sich antiparallel zu dem Strahl 45 ausbreitet. Nach Verlassen des 180°-Prismas 53 durchsetzt der Strahl 59 den Luftspalt 49 und tritt in ein 90°-Prisma 61 mit einer Spiegelfläche 63 ein, an welcher er um weitere 90° nach links abgelenkt wird und nach nochmaligem Durchsetzen des Glasblocks 47 als Strahl 11 in der linken Okularanordnung das Strahlvertauschungssystem 1 verläßt.

Die optischen Weglängen, die die beiden Strahlenbündel zwischen ihrem Eintritt als Strahl 5 und Austritt als Strahl 7 bzw. Eintritt als Strahl 9 und Austritt als Strahl 11 in dem Strahlvertauschungssystem zurücklegen, sind im wesentlichen gleich, was durch die beschriebene Anordnung der Glasprismen und Glasblöcke erreicht wird. Die Glasblöcke 21 und 47 werden hierbei von beiden Strahlenbündeln durchsetzt, während der Glasblock 22 lediglich von dem Strahlenbündel durchsetzt wird, welches als Strahl 5 in das Strahlvertauschungssystem 1 eintritt und als Strahl 7 aus diesem wieder austritt. Letzterer Glasblock 22 dient hierbei zur Kompensation der optischen Weglänge, den das andere Strahlenbündel zusätzlich durchlaufen muss, da es sich als Strahl 55 über eine gewisse Strecke entgegengesetzt zur ursprünglichen Richtung des Strahls 9 von unten nach oben verläuft, während das Strahlenbündel, das als Strahl 5 eintritt, sich nie in eine Richtung entgegen der ursprünglichen Strahlrichtung ausbreitet.

Für einen Feinabgleich der optischen Weglängen, die die beiden Strahlenbündel in dem Strahlvertauschungssystem 1 zurücklegen, sind die Luftspalte 23 und 49 vorgesehen, welche mittels Einstellvorrichtungen 65 änderbar sind, die das 180°-Prisma 25 an den Glasblock 22 bzw. das 180°-Prisma 57 an den Glasblock 47 und das 90°-Prima 15 koppeln.

Wie aus Figur 2a ersichtlich ist, tritt das linke Strahlenbündel 5 als paralleles Strahlenbündel in die Tubuslinse 13 der linken Okularanordnung ein und wird durch diese in ein konvergentes Strahlenbündel umgeformt, so daß in einer Zwischenbildebene 69 der rechten Stereokanals ein scharfes Zwischenbild in der Okular-Zwischenbildebene (69) entsteht. Die Zwischenbildebene 69 ist im Fokus der Tubuslinse 13 mit einem Abstand d1 angeordnet, der durch den Brechungsindex des für die Prismen 15, 25, 35 und die Glasblöcke 21, 22 verwendeten Glases, deren geometrische Abmessungen sowie die Abmessungen der durchlaufenen Luftstrecken bestimmt ist.

Soll nun das Stereo-Mikroskop in dem anderen Beobachtungsmodus eingesetzt werden, bei dem die Ophthalmoskopierlinse aus dem Strahlengang entfernt ist und das zu betrachtende Objekt in der Objektebene des Mikroskops angeordnet ist, so ist eine Strahlvertauschung durch das Strahlvertauschungssystem 1 nicht mehr notwendig, so daß dieses aus dem Strahlengang zu entfernen ist. Andererseits würde dann das in der Objektebene angeordnete Objekt ohne Umkehrprisma von dem Beobachter höhen- und seitenverkehrt wahrgenommen werden. Für die höhen- und seitenrichtige Darstellung des in der Objektebene angeordneten Objekts, wird deshalb statt des Strahlvertauschungssystems 1 in diesem Beobachtungsmodus in dem Strahlengang ein Bildumkehrsystem 73 angeordnet. Dieses wirkt derart, daß das in der linken Okularanordnung verlaufende Strahlenbündel 5 in der linken Okularanordnung verbleibt und als Strahlenbündel 7' in einer Zwischenbildebene 71 der linken Okularanordnung fokussiert wird, wobei auch das in der rechten Okularanordnung verlaufende Strahlenbündel 9 in dieser verbleibt und dort in einer entsprechenden Zwischenbildebene fokussiert wird. Hierzu umfaßt das Bildumkehrsystem 73 jeweils ein Schmidt-Pechan-Prisma mit Dachkante 74 für sowohl die linke als auch die rechte Okularanordnung.

Die geometrischen Abmessungen des Schmidt-Pechan-Prismas 74 sowie der Brechungsindex des für dieses verwendeten Glases bestimmen einen Abstand d2 zwischen der Tubuslinse 13 der linken Okularanordnung und der Zwischenbildebene 71 in dem zweiten Beobachtungsmodus. Durch entsprechende Einstellung der Einstellvorrichtungen 65 der Strahlvertauschungssystem 1 ist es möglich, daß die Abstände d1 und d2 gleich sind, so daß bei einem Wechsel des Beobachtungsmodus eine Nachstellung der Okularanordnung zur Erzielung einer scharfen Abbildung nicht notwendig ist.

Entsprechendes gilt für die Abstände, unter denen eine Zwischenbildebene des rechten Strahlenbündels 9 von der Tubuslinse 39 angeordnet ist, wobei die zugehörigen Strahlengänge in den Figuren 2a und 2b der Übersichtlichkeit halber nicht dargestellt sind.

Im folgenden werden Varianten des in den Figuren 1 und 2 dargestellten Stereo-Mikroskops erläutert. Hinsichtlich ihres Aufbaus und ihrer Funktion einander entsprechende Komponenten sind mit den Bezugszahlen aus den Figuren 1 und 2 bezeichnet, jedoch zur Unterscheidung mit einem zusätzlichen Buchstaben versehen. Zur Erläuterung wird auf die gesamte vorangehende Beschreibung Bezug genommen.

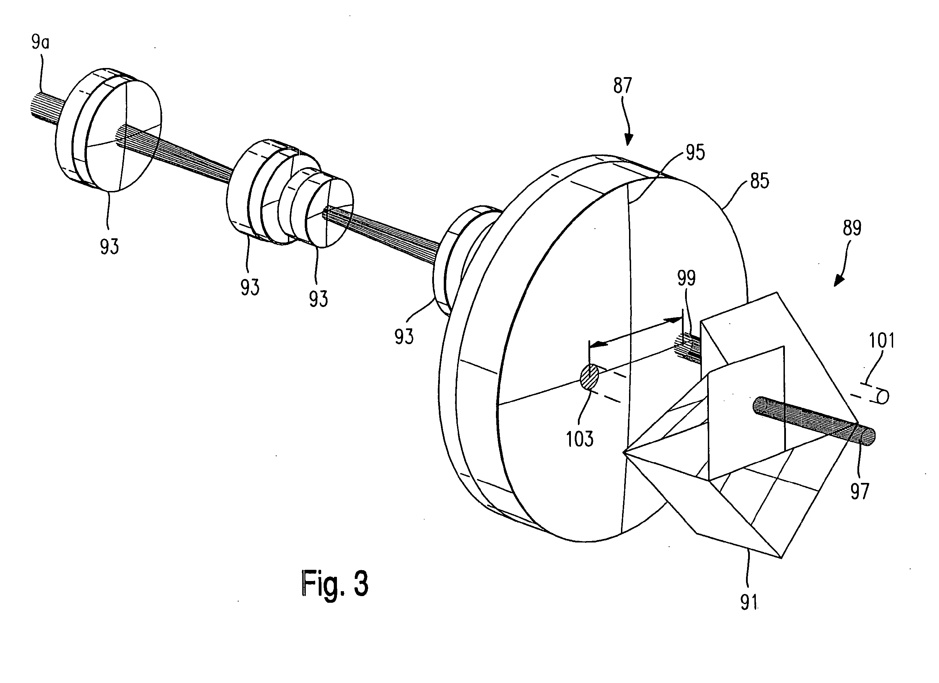

Eine in Figur 3 gezeigte weitere Ausführungsform der Erfindung zeigt eine perspektivische Teilansicht eines Stereo-Mikroskops in dem oben beschriebenen ersten Beobachtungsmodus, d.h. zur Beobachtung eines höhen- und seitenverkehrten Zwischenbilds in der Objektebene des Mikroskops.

Hierzu ist vor einer Eintrittslinse 85 einer Objektivanordnung 87 des Mikroskops ein Strahlvertauschungs- und Bildumkehrsystem 89 aus zwei Porro-Prismen zweiter Art angeordnet, von denen der Übersichtlichkeit halber nur ein Porro-Prisma 91 in Figur 3 dargestellt ist. Zur Änderung der Vergrößerung umfaßt die Objektivanordnung 87 ferner noch ein Zoomsystem mit mehreren Linsen 93. Das Porro-Prisma zweiter Art 91 versetzt ein bezüglich einer Mittelebene 95 der Objektivanordnung 87 links angeordnetes und von der Objektebene her kommendes und auf die Eintrittslinse 85 gerichtetes Strahlenbündel 97 parallel und führt es der Eintrittslinse 85 rechts von der Mittelebene 95 als Strahlenbündel 99 zu. Entsprechend versetzt das zweite in Figur 3 nicht dargestellte Porro-Prisma zweiter Art des Strahlvertauschungs- und Bildumkehrsystems 89 ein von der Objektebene her kommendes und bezüglich der Mittelebene 95 rechts auf die Eintrittslinse 85 gerichtetes Strahlenbündel 101 parallel über die Mittelebene 95 nach links und führt es der Objektivanordnung 87 als Strahlenbündel 103 links zu. Der Verlauf der Strahlenbündel 101 und 103 ist in Figur 3 durch gestrichelte Linien lediglich angedeutet.

Durch die der Objektivanordnung nachfolgende Strahlführung des Mikroskops wird das Strahlenbündel 99 in die rechte Okularanordnung des Mikroskops und entsprechend das Strahlenbündel 103 in die linke Okularanordnung des Mikroskops abgebildet.

Das Strahlvertauschungs- und Bildumkehrsystem 89 umfaßt dabei zwei identische Porro-Prismen zweiter Art, welche so ineinandergefügt sind, daß die Spiegelflächen der kleinen 90°-Prismen Rücken an Rücken aneinanderliegen.

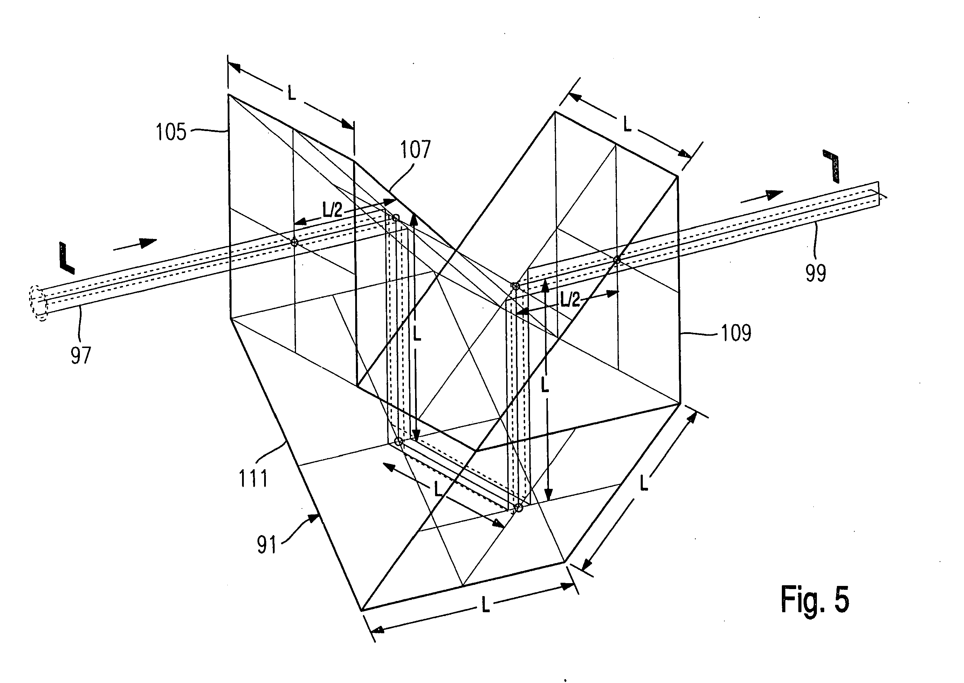

In Figur 5 ist der Strahlverlauf durch das Porro-Prisma zweiter Art 91 vergrößert dargestellt. Dieses Porro-Prisma 91 kann man sich zusammengesetzt denken aus zwei kleinen 90°-Prismen 105 und 109 sowie einem großen 180°-Prisma 111. Die beiden 90°-Prismen 105 und 109 weisen dabei eine kurze Kante der Länge L auf. Der Strahl 97 tritt in das erste 90°-Prisma 105 ein und durchläuft darin eine Strecke von L/2 bis zu seiner Reflexion um 90° an einer Spiegelfläche 107 des Prismas 105. Daraufhin durchläuft der Strahl noch eine Strecke von L/2, bis er in das 180°-Prisma 111 übertritt, worin er wiederum eine Strecke von L/2 bis zu seiner ersten Reflexion um 90° darin zurücklegt. Hiernach durchläuft der Strahl in dem 180°-Prisma 111 eine Strecke der Länge L bis zu seiner zweiten Reflexion darin um weitere 90°, woraufhin der Strahl eine weitere Strecke von L/2 durchläuft, bis er das 180°-Prisma 111 verläßt und in das zweite 90°-Prisma 109 eintritt. Darin durchläuft der Strahl wiederum eine Strecke von L/2 bis zu seiner Reflexion um 90° an der Spiegelfläche des 90°-Prismas 109, welches er nach einer weiteren Strecke von L/2 als Strahl 99 verläßt. Es ist ersichtlich, daß der Strahl in dem Prisma 91 somit eine gesamte geometrische Länge von 4 L zurücklegt.

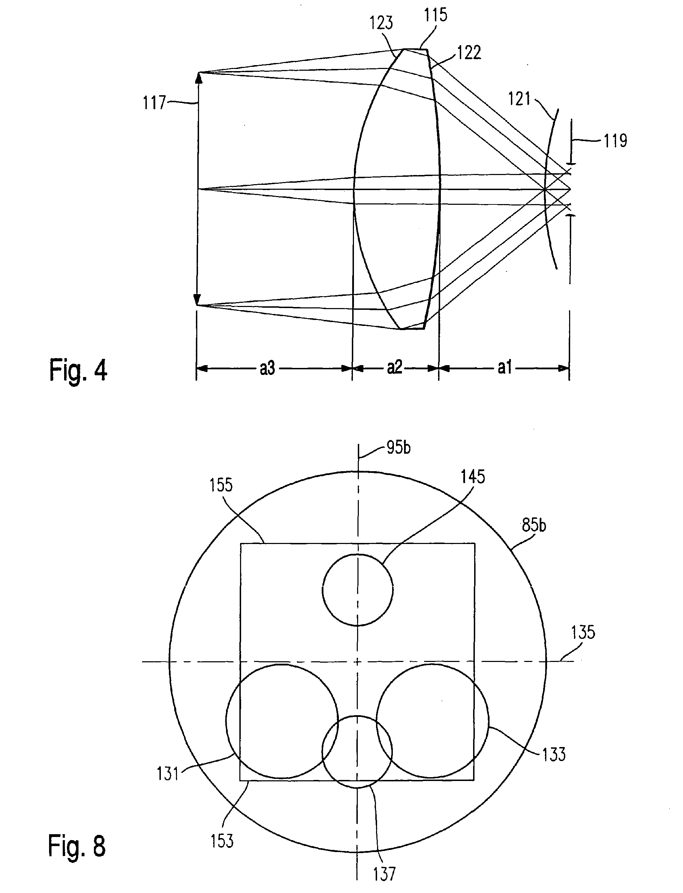

In Figur 4 ist eine zur Verwendung mit dem Stereo-Mikroskop vorgesehene Ophthalmoskopierlinse 115 dargestellt, welche vor einem Auge eines Patienten zur Anordnung kommt, um ein Abbild eines Augenfundus in einer Objektebene 117 des Stereo-Mikroskops zu erzeugen. Von dem zu untersuchenden Auge ist in Figur 4 lediglich schematisch eine Pupillenöffnung 119 und eine Oberfläche einer Cornea 121 dargestellt. Die Linse 115 hat eine dem Auge zu weisende Linsenfläche 122 mit einem Krümmungsradius von 42,474 mm und eine der Objektebene 117 zu weisende asphärische Linsenfläche 123 mit einem Krümmungsradius von 11,372 mm.

Die dem Auge zu weisende Linsenfläche 121 weist in ihrem Zentrum von der Pupille 119 des Auges einen Abstand a1 von etwa 8,5 mm auf. Die beiden Linsenflächen 121 und 123 der Linse 115 sind an ihren Zentren mit einem Abstand a2 von 5,6 mm voneinander angeordnet, während die Linsenfläche 123 von der Objektebene 117 mit einem Abstand a3 von 9,99 mm angeordnet ist. Somit weist die Pupille 119 von der Zwischenbildebene einen Abstand von etwa 24,09 mm auf, wobei die Fläche der Cornea 21 von der Pupillenebene typischerweise etwa 1 mm entfernt ist.

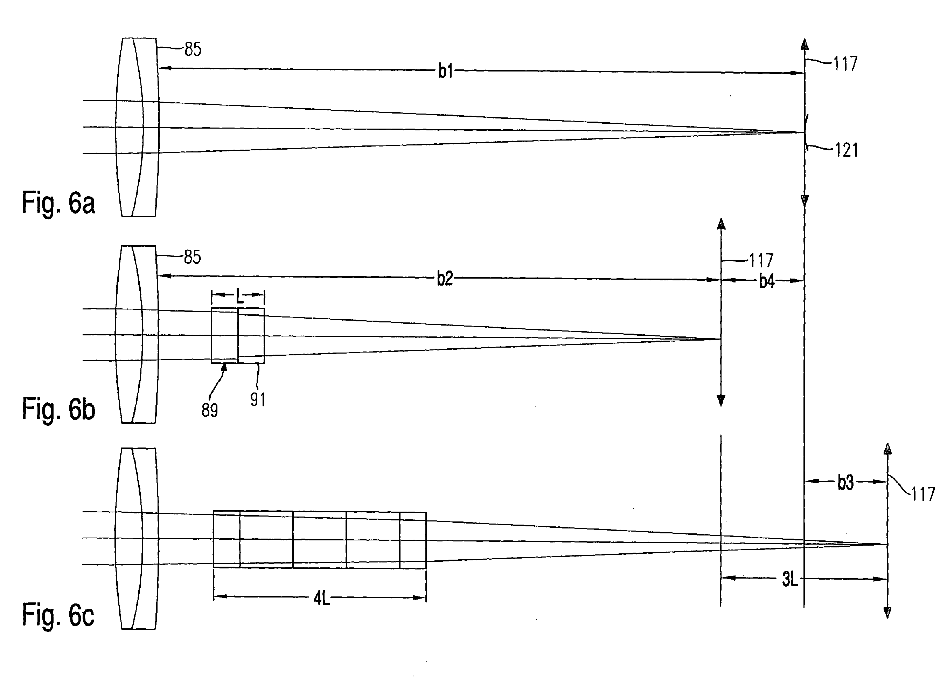

In Figur 6a ist der Strahlenverlauf und die Anordnung der Objektebene 117 des Stereo-Mikroskops in dem zweiten Beobachtungsmodus dargestellt, d.h. in dem Modus, in dem das Strahlvertauschungs- und Bildumkehrsystem 89 aus dem Strahlengang entfernt ist und in dem mit dem Mikroskop die Cornea des zu operierenden Auges betrachtet wird. Es ist somit auch die Cornea 121 in der Objektebene 117 angeordnet. Der Abstand zwischen der Eintrittslinse 85 und der Objektebene 117 beträgt b1.

In Figur 6b ist die Anordnung in dem ersten Beobachtungsmodus dargestellt, in dem das Strahlvertauschungs- und Bildumkehrsystem 89 vor der Eintrittslinse 85 angeordnet ist, um das von der Ophthalmoskopierlinse 115 erzeugte höhen- und seitenverkehrte, pseudostereoskopische Zwischenbild des Augenfundus in der Objektebene 117 des Mikroskops zu beobachten.

Dieses Zwischenbild befindet sich nach Figur 4 im Abstand B4 = a1 + a2 + a3 vor der Augenpupille. Auf diese Zwischenbildebene muß also im ersten Beobachtungsmodus mit dem Mikroskop fokussiert werden, sie stellt also im ersten Beobachtungsmodus die neue Objektebene für das Mikroskop dar.

Aufgrund der erfindungsgemäßen Ausführung des Porro-Prismas zweiter Art 91 kann nun die mit dem Mikroskop beobachtete Objektebene 117 im ersten Beobachtungsmodus exakt in einen Abstand b2 von der Eintrittslinse 85 verschoben werden, so daß die Differenz b1 - b2 = b4 ist. Somit muss beim Wechsel zwischen den beiden Beobachtungsmodi für eine optimale Bildschärfe nicht nachfokussiert werden.

Zur Erläuterung dieser Bedingung für die Differenz b1 - b2 ist in Figur 6c der Strahlengang in dem ersten Beobachtungsmodus derart dargestellt, daß der Strahlenverlauf durch das Porro-Prisma 2. Art 91 entfaltet gezeigt ist.

Einerseits wird nun durch einen Glasblock der Länge 4L aus optischen Gründen die Objektebene 117 um b3 = 4L * (n-1)/n verschoben.

Andererseits wird die Objektebene durch die Faltung des Strahlengangs aus mechanischen Gründen um 3L in die entgegengesetzte Richtung verschoben.

Für die Verschiebung der Objektebene um b4 = 3L - b3 in die von der Ophthalmoskopierlinse gegebene Zwischenbildlage des Augenfundus sind also sowohl die Brechzahl n vom Prismenglas als auch die Kantenlänge L des Prismas maßgebend.

Beispielhaft soll hier eine Verschiebung um b4 = 25.0 mm angegeben werden.

Bei einer Bemessung der Kantenlänge L des Prismas 91 zu 16.84 mm und bei Verwendung eines Glases mit der Typenbezeichnung N-SK2 der Firma Schott mit einem Brechungsindex n = 1.60994 ergibt sich für den Abstand B3 ein Wert von 25.52 mm, so daß sich mit 3L = 50.52 mm eine Verschiebung der Objektebene b4 = 3L - b3 von 25.0 mm ergibt.

Durch Änderung des Glases und der Kantenlänge des Prismas kann dieser Wert in einer für die praktischen Erfordernisse ausreichende Weise variiert werden.

Dies bedeutet, daß beim Wechsel von dem zweiten Beobachtungsmodus, bei dem das Mikroskop auf die Cornea des Auges scharf eingestellt ist, in den ersten Beobachtungsmodus, bei dem die Ophthalmoskopierlinse 115 vor dem Auge angeordnet ist, um das Abbild des Augenfundus zu beobachten, im Wesentlichen keine Änderung des Abstands zwischen der Objektivlinse 85 und dem Auge notwendig ist, was für den Operateur eine große Erleichterung darstellt.

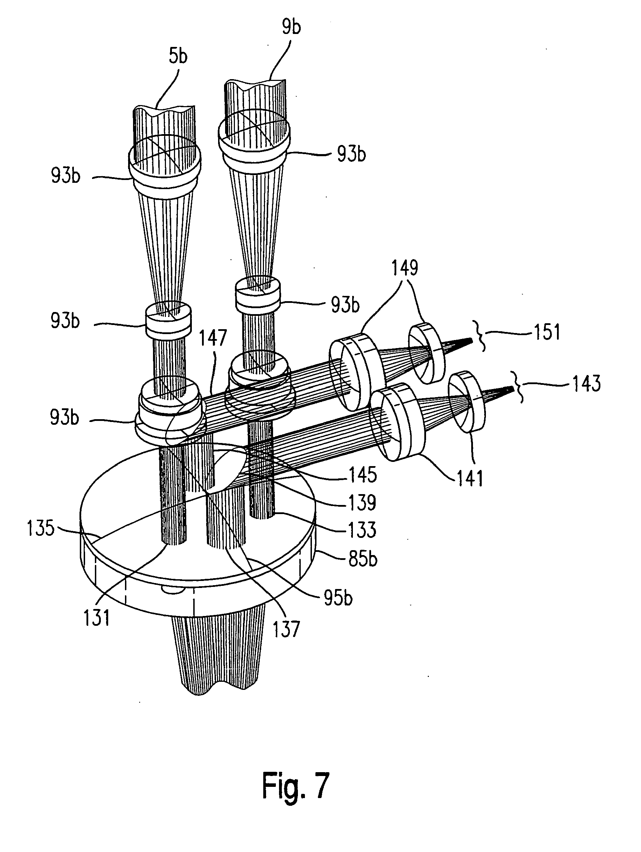

In den Figuren 7 bis 9 ist eine Variante der vorangehend erläuterten Ausführungsform dargestellt, die sich von dieser hauptsächlich dadurch unterscheidet, daß das Stereo-Mikroskop nicht nur für einen Beobachter vorgesehen ist, d.h. mit einem Paar Okularanordnungen versehen ist, sondern daß das Mikroskop vielmehr für zwei Beobachter vorgesehen ist, weshalb zwei Paare von stereoskopischen Beobachtungskanälen mit jeweils zwei Okularen vorgesehen sind.

Figur 7 ist eine räumliche Teildarstellung des Strahlenverlaufs dieses Mikroskops, welche zeigt, wie die entsprechenden Strahlenbündel an die beiden Paare von Okularanordnungen zugeführt werden.

In Figur 7 ist ein Strahlenbündel 5b gezeigt, welches einem linken Okular eines ersten Beobachters zugeführt wird, sowie ein Strahlenbündel 9b, welches einem rechten Okular des ersten Beobachters zugeführt wird. Das Strahlenbündel 5b tritt als Strahlenbündel 131 links von einer Mittelebene 95b einer Objektivlinse 85b des Mikroskops aus, wobei es in ein Zoomsystem aus Linsen 93b eintritt und dieses als Strahlenbündel 5b verläßt. Entsprechend tritt rechts der Mittelebene 95b ein Strahlenbündel 133 aus der Objektivlinse 85b aus, welches ebenfalls Zoomlinsen 93b des rechten Stereokanals des ersten Mitbenutzers durchläuft und als Strahlenbündel 9b dann in das rechte Okular des ersten Beobachters eintritt.

Senkrecht zu der Mittelebene 95b ist auf der Eintrittslinse 85 in Figur 7 eine Horizontalebene 135 dargestellt, so daß die Mittelebene 95b und die Horizontalebene 135 die Eintrittslinse 85b in gleichförmige Quadranten aufteilen.

Ein unterhalb der Horizontalebene 95 zentral auf der Zentralebene 95b austretendes Strahlenbündel 137 trifft auf einen unter 45° zur Ausbreitungsrichtung des Strahlenbündels 137 angeordneten Spiegel 139, welcher das Strahlenbündel 137 um 90° zu seiner ursprünglichen Strahlrichtung reflektiert. Der Strahl 137 durchläuft daraufhin ein Galileisystem aus Linsen 141 und wird als Strahlenbündel 143 einem linken Okular eines zweiten Beobachters zugeführt. Ein oberhalb der Horizontalebene 135 und zentral auf der Mittelebene 95b aus der Eintrittslinse 85b austretendes Strahlenbündel 145 wird an einem parallel zu dem Spiegel 139 angeordneten weiteren Spiegel 147 ebenfalls um 90° reflektiert und durchsetzt eine weitere Galileianordnung aus Linsen 149, welche parallel zu der Galileianordnung aus den Linsen 141 für den Strahl 137 angeordnet ist, worauf das Strahlenbündel 145 dann als Strahl 151 einem rechten Okular des zweiten Beobachters zugeführt wird.

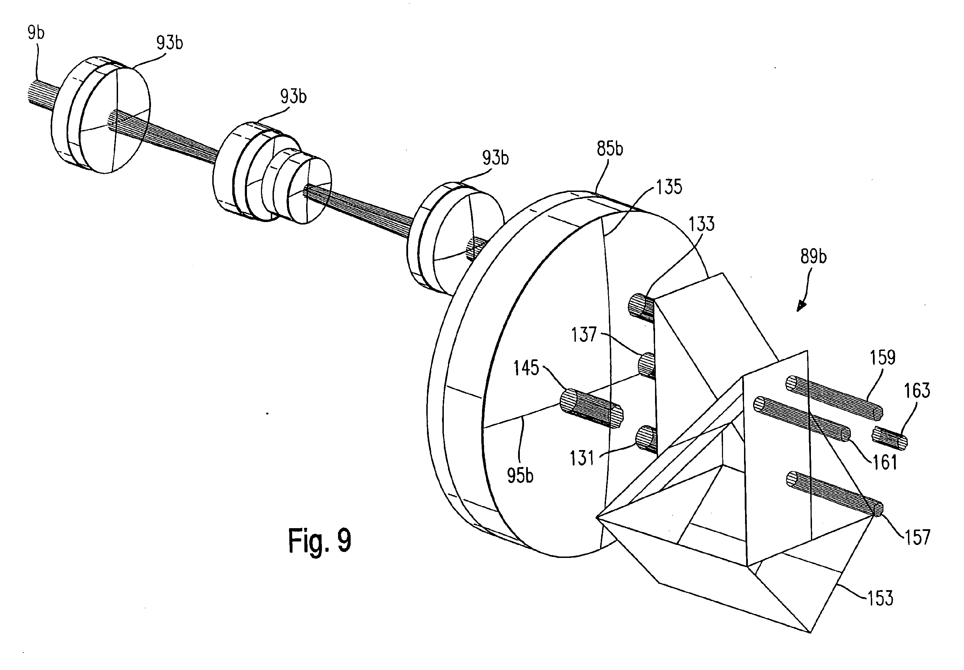

In Figur 8 sind Projektionen der Querschnitte der Strahlenbündel 131, 133, 137 und 145 in Draufsicht auf die von der Objektebene wegweisende Linsenfläche der Linse 85b nochmals im Querschnitt dargestellt, wobei die Bündel 131 und 133 für die bei hoher Vergrößerung auftretenden großen Bündelquerschnitte dargestellt sind.

Aus der perspektivischen Teildarstellung der Figur 9 sind die Strahlenbündel 131, 133, 137 und 145 zu entnehmen, wie sie in die Eintrittslinse 85b des Objekts eintreten. Von den austretenden Strahlenbündeln ist lediglich das Strahlenbündel mit zugehörigen Zoomlinsen 93b gezeigt, welches sich als Strahlenbündel 9b in das rechte Okular des ersten Beobachters fortsetzt. Vor ihrem Eintritt in die Eintrittslinse 85b haben die Strahlenbündel 131, 133, 137 und 145 ein Strahlvertauschungs- und Bildumkehrsystem 89b durchlaufen, welches für die Betrachtung eines höhen- und seitenverkehrten Zwischenbilds in der Objektebene sowohl die notwendige Strahlvertauschung als auch die Bildumkehr bereitstellt.

Das Strahlvertauschungs- und Bildumkehrsystem 89b umfaßt zwei Porro-Prismen zweiter Art 153 und 155. In der perspektivischen Darstellung der Figur 9 ist lediglich das Prisma 153 dargestellt, während aus der Figur 8 Projektionen beider Prismen 153 und 155 sichtbar sind.

Das Prisma 153 bewerkstelligt die Strahlvertauschung und Bildumkehr für die beiden den Okularen des ersten Beobachters zugeführten Strahlenbündel 5b und 9b. Hierzu wird durch das Prisma 153 ein von der Objektebene her kommendes und links der Zentralebene 95b auf die Eintrittslinse 85b gerichtetes Strahlenbündel 157 durch das Prisma 153 derart versetzt, daß es das Prisma 153 als das Strahlenbündel 133 verläßt, welches rechts der Mittelebene 95b in das Objektiv eintritt und als Strahlenbündel 9b für das rechte Auge des ersten Beobachters bestimmt ist. Entsprechend tritt ein rechts der Zentralebene 95b auf die Eintrittslinse 85b gerichtetes Strahlenbündel 159 in das Prisma 153 ein und aus diesem als Strahlenbündel 131 wieder aus, welches für das linke Auge des ersten Beobachters bestimmt ist.

Weiterhin tritt in das Prisma 153 ein Strahlenbündel 161 ein, welches zentral auf die Mittelebene 95b und oberhalb der Horizontalebene 135 auf die Eintrittslinse 85b gerichtet ist. Dieses Strahlenbündel 161 wird durch das Prisma 153 derart versetzt, daß es als das Strahlenbündel 137 zentral auf der Mittelebene 95b und unterhalb der Horizontalebene 135 in das Objektiv einfällt, wie dies auch aus Figur 8 ersichtlich ist. Wie vorangehend bereits erläutert, ist das Strahlenbündel 137 für das linke Auge des zweiten Beobachters bestimmt.

In das zweite Prisma 155 des Strahlvertauschungs- und Bildumkehrsystems 89b tritt lediglich ein Strahlenbündel 163 ein, dessen räumliche Anordnung bezüglich der anderen Strahlenbündel 157, 159 und 161 aus Figur 9 ersichtlich ist. Das Strahlenbündel 163 ist bezüglich der Zentralebene 95b mittig und unterhalb der Horizontalebene 135 auf die Eintrittslinse 85b des Objektivs gerichtet. Durch das Prisma 155 wird das Strahlenbündel 163 derart versetzt, daß es als das Strahlenbündel 145 bezüglich der Zentralebene 95b mittig und oberhalb der Horizontalebene 135 in das Objektiv eintritt, wie dies in Figur 9 ebenfalls andeutungsweise dargestellt ist.

In der Darstellung der Figur 8 sind die projizierten Austrittsflächen der beiden Prismen 153 und 155 dargestellt. Aus der Figur 8 ist damit ersichtlich, daß aus dem Porro-Prisma zweiter Art 153 die beiden für das linke und das rechte Auge des ersten Beobachters bestimmten Strahlenbündel 131 und 133 sowie das für das rechte Auge des zweiten Beobachters bestimmte Strahlenbündel 137 austreten. Aus dem Prisma 155 tritt lediglich das für das linke Auge des zweiten Beobachters bestimmte Strahlenbündel 145 aus.

Mit dieser Anordnung ist ein vor dem Objektiv des Mikroskops anordenbares Strahlvertauschungs- und Bildumkehrsystem realisiert, welches vergleichsweise wenig Bauraum einnimmt und damit den Zugang zum Operationsfeld verhältnismäßig wenig stört. Zudem erfolgt die Strahlvertauschung und Bildumkehr für den ersten Beobachter durch ein integrales Prisma, so daß ein Problem der Justierung der Bildlagen für das linke und das rechte Auge relativ zueinander für diesen Beobachter nicht auftritt. Allerdings sind auch zwei identische Porro-Prismen zweiter Art mit solcher Präzision ineinandersteckbar und relativ zueinander justierbar, daß auch für den zweiten Benutzer, dessen beide Stereokanäle beide Prismen benutzen, Bildlagenprobleme weitgehend vermeidbar sind.

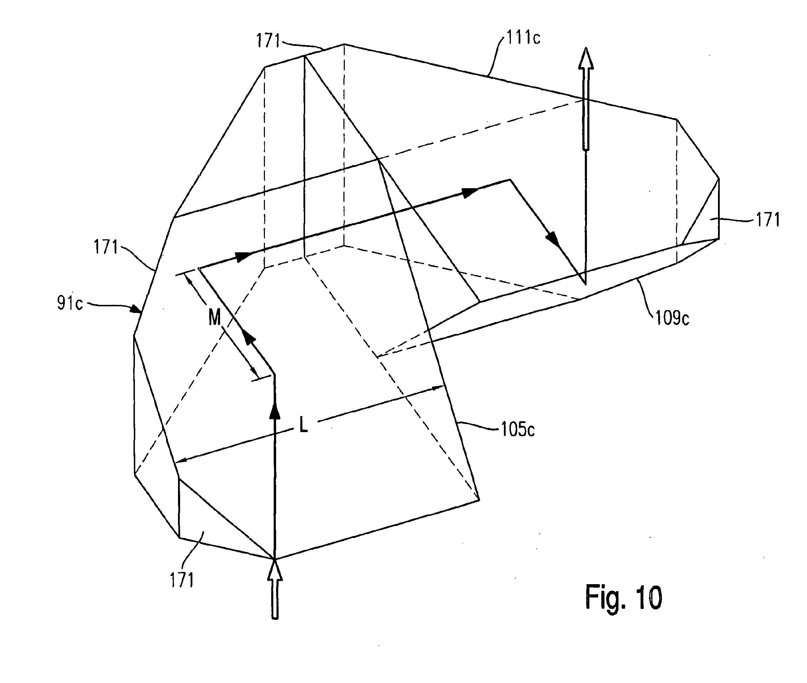

In Figur 10 ist ein an sich bekanntes "verkürztes" Porro-Prisma zweiter Art 91c dargestellt. Auch dieses Prisma kann man sich zusammengesetzt denken aus zwei 90°-Prismen 105c und 109c und einem 180°-Prisma 111c. Im Unterschied zu dem in Figur 5 gezeigten Porro-Prisma zweiter Art ist bei dem Prisma 91c der Figur 10 das 180°-Prisma 111c in seiner Scheitelhöhe verkürzt, so daß dessen reflektierende Flächen näher an die 90°-Prismen 105c und 109c herangerückt sind. Zudem sind bei sämtlichen Teilprismen 105c, 109c, 111c des Prismas 91c Kanten 171 gebrochen, welche von dem zu spiegelnden Strahlenbündel nicht durchsetzt werden. Wie aus Figur 10 ersichtlich ist, legt ein Zentralstrahl zwischen seiner Reflexion an dem 90°-Prisma 105c und seiner ersten Reflexion an dem 180°-Prisma 111c eine Strecke M zurück, welche sich berechnet zu M = 0,71 L, wobei L wieder eine Kantenlänge des 90°-Prismas 105c, 109c darstellt.

Somit weist die in dem Prisma 91c zurückgelegte Strecke eines Strahls einen Wert von 3,42 L auf, was im Vergleich zu dem Wert von 4 L für das Prisma der Figur 5 eine verkürzte optische Weglänge bedeutet. Gleichzeitig wirkt das Prisma 91c ebenfalls als Strahlvertauschungs- und Bildumkehrsystem, welches in einem Stereo-Mikroskop zur Betrachtung eines höhen- und seitenverkehrten Zwischenbildes einsetzbar ist.

Das verkürzte Porro-Prisma zweiter Art kann an verschiedenen Stellen des Mikroskops eingesetzt werden. Hierbei kommt eine Anordnung vor dem Objektiv in Betracht, wie das für die normalen Porro-Prismen zweiter Art bereits in Zusammenhang mit den in den Figuren 3 und 9 dargestellten Ausführungsformen erläutert wurde.

Weiterhin ist das verkürzte Porro-Prisma zweiter Art zur Strahlvertauschung und Bildumkehr im Strahlengang zwischen dem Objektiv und den Okularen einsetzbar, also bei einem Vergleich mit der eingangs bereits erläuterten Figur 11 zwischen den Elementen 909 und 913. Hierbei ist schließlich eine Anordnung zwischen einem Vergrößerungs-Änderungssystem und den Okularen möglich (zwischen den Komponenten 911 und 913 in Figur 11), besonders bevorzugt ist aber eine Anordnung zwischen dem Objektiv bzw. der Sammellinse des Objektivs und dem Vergrößerungs-Änderungssystem (zwischen den Komponenten 909 und 911 in Figur 11). Diese Anordnung ist deshalb besonders bevorzugt, weil dort eine Störung des erzeugten Bildes, wie etwa Vignettierung, in vergleichsweise geringem Umfang auftritt.

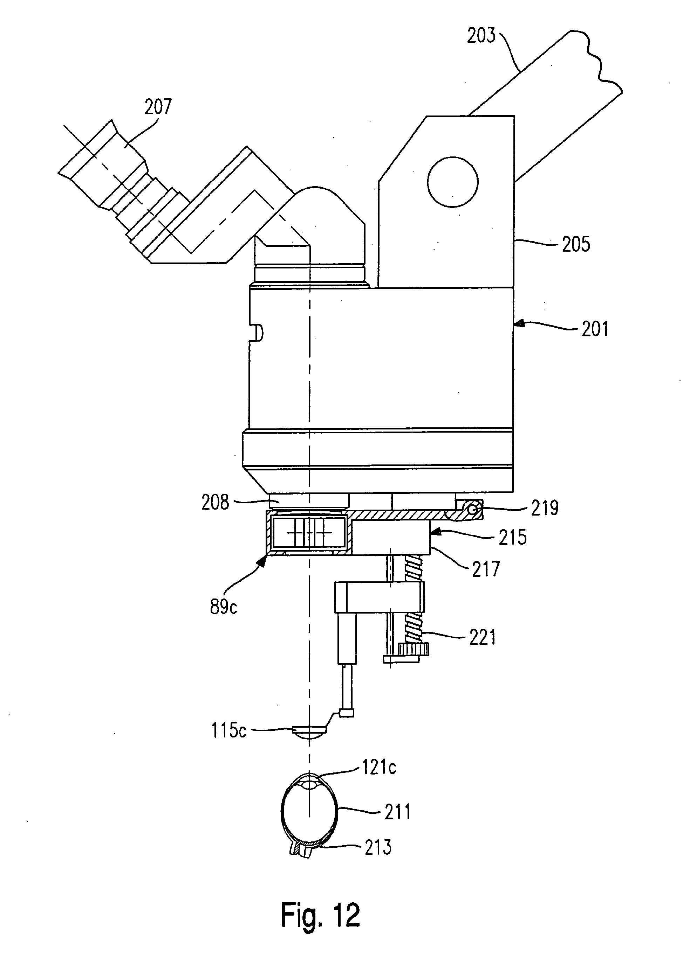

In Figur 12 ist eine Seitenansicht eines Stereo-Mikroskops 201 dargestellt, welches mit einem Strahlvertauschungs- und Bildumkehrsystem 89c versehen ist. Ein Mikroskopkörper 205 ist an einem Stativ 203 verschenkbar gehaltert und trägt ein Paar Okulare 207 und ein Objektiv 208, wobei die in den Okularen 207, dem Objektiv 208 sowie weiter in dem Mikroskopkörper 205 enthaltenen Linsensysteme in Figur 12 nicht dargestellt sind. Diese Linsensysteme können einen Aufbau aufweisen, wie er in den Figuren 3 bis 11 erläutert wurde, wobei auch andere Linsenanordnungen möglich sind. Das Stereo-Mikroskop 201 ist zur Beobachtung eines Patientenauges 211 vorgesehen, und zwar soll ein Augenfundus 213 durch eine Cornea 121c des Auges beobachtet werden. Hierzu weist das Mikroskop 201 ein an dem Mikroskopkörper 205 gehaltertes Vorsatzsystem 215 auf. Als optische Komponenten umfaßt das Vorsatzsystem 215 das Strahlvertauschungs- und Bildumkehrsystem 89c, welches vor dem Objektiv 208 zur Anordnung kommt, und eine Ophtalmoskopierlinse 115c, welche zwischen dem Strahlvertauschungs- und Bildumkehrsystem 89c und der Cornea 121c zur Anordnung kommt. Das Strahlvertauschungs- und Bildumkehrsystem 89c und die Ophtalmoskopierlinse 115c sind an einer gemeinsamen Halterung 217 befestigt, welche wiederum an dem Mikroskopkörper 205 gehaltert ist. Hierbei ist die Halterung 217 um eine Achse 219 schwenkbar an dem Mikroskopkörper 205 gehaltert, so daß das optische System aus Strahlvertauschungs- und Bildumkehrsystem 89c und Ophtalmoskopierlinse 115 auf einfache Weise in den Strahlengang des Mikroskops einschwenkbar und aus diesem wieder entfernbar ist. Im Hinblick auf eine einfache Abstimmung des von dem Mikroskop 201 erzeugten Bildes des Augenfundus 213 ist der Abstand zwischen Ophtalmoskopierlinse 115 und dem Strahlvertauschungs- und Bildumkehrsystem 89c über einen Spindeltrieb 221 änderbar.

In dem vorangehend beschriebenen Ausführungsbeispiel der Figur 12 sind das Strahlvertauschungs- und Bildumkehrsystem und die Ophtalmoskopierlinse 115 gemeinsam in den Strahlengang des Mikroskops einschwenkbar gehaltert. Es ist jedoch ebenfalls möglich, die Ophtalmoskopierlinse separat von dem Mikroskop, beispielsweise an einem Stativ, zu haltern und das Strahlvertauschungs- und Bildumkehrsystem schwenkbar an dem Mikroskop zu haltern. Ebenso ist es möglich, von der in Figur 12 gezeigten schwenkbaren Halterung abzuweichen und beispielsweise eine oder mehrere anders orientierte Schwenkachsen vorzusehen. Ferner ist es möglich, das Vorsatzsystem beispielsweise an einem Schlitten zu haltern, um es in den Strahlengang hineinzuschieben und aus diesem auch wieder herauszuschieben.

Das in Figur 1 gezeigte Strahlvertauschungssystem ist bei der dort beschriebenen Ausführungsform im Strahlengang der Okularanordnungen des Stereo-Mikroskops angeordnet. Allerdings ist dieses Strahlvertauschungssystem auch an jeder anderen Stelle des Strahlengangs des Stereo-Mikroskops einsetzbar, um die gewünschte Wirkung, nämlich eine Strahlvertauschung unter Beibehaltung der Bildorientierung zu erzielen.

Ferner ist der Einsatz dieses Strahlvertauschungssystems, also der in Zusammenhang mit Figur 1 beschriebenen Anordnung, von zwei 90°-Spiegeln und einem 180°-Doppelspiegel pro Strahlengang nicht auf die Anwendung in Stereo-Mikroskopen beschränkt. Es ist die Verwendung dieses Strahlvertauschungssystems vielmehr für jeden anderen Bereich der Optik vorgesehen, in dem das Problem einer Strahlvertauschung unter Beibehalten der Bildorientierung auftritt.

In den vorangehenden Ausführungsformen wurden jeweils die Verwendung einer Ophthalmoskopierlinse zur Erzeugung eines höhen- und seitenverkehrten Zwischenbilds eines Augenfundus beschrieben, um das Zwischenbild in der Objektivebene des Mikroskops zu erzeugen. Allerdings ist das beschriebene Mikroskop für jede andere Anwendung vorgesehen, bei der das Problem der Betrachtung eines höhen- und seitenverkehrten Zwischenbilds in der Objektebene des Mikroskops auftritt. Dies können beispielsweise andere chirurgische Anwendungen oder jeglicher anderer Einsatz sein, für den die Verwendung eines Stereo-Mikroskops in Betracht kommt.

Ferner offenbart die vorliegende Anmeldung das Folgende:
1. Eine Stereo-Mikroskopieanordnung zur Betrachtung eines Objekts oder eines von einem Objekt erzeugten Zwischenbilds, insbesondere in der Mikrochirurgie, umfassend eine Objektivanordnung mit einer Objektebene zur Anordnung des zu betrachtenden Objekts bzw. Zwischenbilds, und ein Strahlvertauschungs- und Bildumkehrsystem, das ein von der Objektebene nach links in Richtung zu der Objektivanordnung gerichtetes Strahlenbündel der Objektivanordnung rechts zuführt, das ein von der Objektebene nach rechts in Richtung zu der Objektivanordnung gerichtetes Strahlenbündel der Objektivanordnung links zuführt, und das Bildorientierungen der beiden Strahlenbündel jeweils umkehrt,
   wobei das Strahlvertauschungs- und Bildumkehrsystem wenigstens ein Porro-Prisma zweiter Art umfaßt.
2. Eine Stereo-Mikroskopieanordnung nach Item 1, wobei für ein jedes der beiden Strahlenbündel ein separates Porro-Prisma zweiter Art vorgesehen ist und wobei die beiden Porro-Prismen zweiter Art zu einer symmetrischen Anordnung ineinandergefügt sind.
3. Eine Stereo-Mikroskopieanordnung nach Item 1, wobei für einen ersten Beobachter zwei Okularanordnungen vorgesehen sind, denen die der Objektivanordnung links und rechts zugeführten Strahlenbündel zugeführt werden, wobei für einen zweiten Beobachter zwei weitere Okularanordnungen vorgesehen sind, denen der Objektivanordnung oben und unten zugeführte Strahlenbündel zugeführt werden, wobei ein erstes Porro-Prisma zweiter Art vorgesehen ist, welches die der Objektivanordnung links und rechts zugeführten Strahlenbündel zuführt und welches weiter das eine er der Objektivanordnung oben und unten zugeführte Strahlenbündel zuführt, und wobei ein zweites Porro-Prisma zweiter Art vorgesehen ist, welches das andere der der Objektivanordnung oben und unten zugeführten Strahlenbündel zuführt.
4. Eine Stereo-Mikroskopieanordnung nach Item 1 bis 3, wobei das Strahlvertauschungs- und Bildumkehrsystem in den Strahlengang einschwenkbar oder einschiebbar ist.
5. Eine Stereo-Mikroskopieanordnung nach Item 4, ferner umfassend einen Ophtalmoskopie-Vorsatz zur Erzeugung eines Zwischenbilds eines Augenfundus in der Objektebene der Objektivanordnung, welcher gemeinsam mit dem Strahlvertauschungs- und Bildumkehrsystem in den Strahlengang einschwenkbar bzw. einschiebbar ist.
6. Eine Stereo-Mikroskopieanordnung nach Item 1 bis 3 oder dem ersten Absatz von Item 1, ferner umfassend ein vor der Objektivanordnung anordenbares Ophtalmoskopierlinsensystem zur Erzeugung eines seiten- und höhenverkehrten Zwischenbilds eines Augenfundus in der Objektebene der Objektivanordnung, wobei das Strahlvertauschungs- und Bildumkehrsystem und das Vorsatzlinsensystem aus dem Strahlengang vor der Objektivanordnung entfernbar sind, und wobei geometrische Abmessungen und optische Medien der Strahlvertauschungs- und Bildumkehr-Anordnung und Brennweiten des Vorsatzlinsensystems derart abgestimmt sind, daß bei in dem Strahlengang angeordnetem Strahlvertauschungs- und Bildumkehrsystem und Vorsatzlinsensystem das Zwischenbild des Augenfundus in der Objektebene der Objektivanordnung anordenbar ist und dabei die Objektivanordnung von dem Auge einen ersten Abstand aufweist, und daß
   bei aus dem Strahlengang entfernten Strahlvertauschungs- und Bildumkehrsystem und Vorsatzlinsensystem die Cornea des Auges in der Objektebene der Objektivanordnung anordenbar ist und dabei die Objektivanordnng von dem Auge einen zweiten Abstand aufweist, und daß der erste und der zweite Abstand im wesentlichen gleich sind.
7. Eine Stereo-Mikroskopieanordnung nach Item 1 bis 4 oder dem ersten Absatz von Item 1, wobei das Strahlvertauschungs- und Bildumkehrsystem aus dem Strahlengang vor der Objektivanordnung entfernbar ist,
   wobei bei in dem Strahlengang angeordnetem Strahlvertauschungs- und Bildumkehrsystem die Objektebene der Objektivanordnung von der Objektivanordnung einen dritten Abstand aufweist, wobei bei aus dem Strahlengang entferntem Strahlvertauschungs- und Bildumkehrsystem die Objektebene von der Objektivanordnung einen vierten Abstand aufweist, und wobei eine Differenz zwischen dem dritten und dem vierten Abstand einen vorbestimmten Wert aufweist.
8. Eine Stereo-Mikroskopieanordnung nach Item 7, wobei die Differenz zwischen dem dritten und dem vierten Abstand 15 mm bis 40 mm, vorzugsweise 20 mm bis 30 mm, und weiter bevorzugt 24 mm bis 26 mm beträgt.
9. Eine Stereo-Mikroskopieanordnung zur Betrachtung eines Objekts oder eines von einem Objekt erzeugten Zwischenbilds, insbesondere in der Mikrochirurgie, umfassend eine Objektivanordnung mit einer Objektebene zur Anordnung des zu betrachtenden Objekts bzw. Zwischenbilds, eine linke und eine rechte Okularanordnung, und ein Strahlvertauschungs- und Bildumkehrsystem, das ein in die Objektivanordnung links eintretendes Strahlenbündel der rechten Okularanordnung zuführt, das ein in die Objektivanordnung rechts eintretendes Strahlenbündel der linken Okularanordnung zuführt und das Bildorientierungen der beiden Strahlenbündel jeweils umkehrt,
   wobei das Strahlvertauschungs- und Bildumkehrsystem ein Paar verkürzter Porro-Prismen zweiter Art umfaßt.
10. Eine Stereo-Mikroskopieanordnung nach Item 9 oder dem ersten Absatz von Item 9, ferner umfassend ein zwischen der Objektivanordnung und den beiden Okularanordnungen angeordnetes Vergrößerungsänderungssystem, wobei das Strahlvertauschungs- und Bildumkehrsystem zwischen der Objektivanordnung und dem Vergrößerungsänderungssystem angeordnet ist.
11. Eine Stereo-Mikroskopieanordnung nach Item 9 oder 10, ferner umfassend ein vor der Objektivanordnung anordenbares Vorsatzlinsensystem zur Erzeugung eines seiten- und höhenverkehrten Zwischenbilds eines zu betrachtenden Objekts in der Objektebene der Objektivanordnung.
12. Eine Stereo-Mikroskopieanordnung zur Betrachtung eines Objekts oder eines von einem Objekt erzeugten Zwischenbilds, insbesondere in der Mikrochirurgie, umfassend
   eine Objektivanordnung mit einer Objektebene zur Anordnung des zu betrachtenden Objekts bzw. Zwischenbilds,
   eine linke Okularanordnung, der ein in die Objektivanordnung links eintretendes Strahlenbündel zugeführt wird, und eine rechte Okularanordnung, der ein in die Objektivanordnung rechts eintretendes Strahlenbündel zugeführt wird,
   wobei in den Strahlengängen der Okularanordnungen ein Strahlvertauschungssystem vorgesehen ist, welches das der linken Okularanordnung zugeführte Strahlenbündel in die rechte Okularanordnung spiegelt und welches das der rechten Okularanordnung zugeführte Strahlenbündel in die linke Okularanordnung spiegelt,
   wobei die dem Strahlvertauschungssystem zugeführten Strahlenbündel und aus dem Strahlvertauschungssystem austretende Strahlenbündel jeweils einander gleiche Bildorientierungen aufweisen.
13. Eine Stereo-Mikroskopieanordnung nach Item 12, wobei die linke und die rechte Okularanordnung jeweils einen Bereich mit einem konvergenten Strahlengang aufweisen und wobei das Strahlvertauschungssystem in dem konvergenten Strahlengang angeordnet ist.
14. Eine Stereo-Mikroskopieanordnung nach Item 12 oder 13, wobei das Strahlvertauschungssystem für das der rechten Okularanordnung zugeführte Strahlenbündel und für das der linken Okularanordnung zugeführte Strahlenbündel jeweils im wesentlichen gleiche optische Weglängen bereitstellt.
15. Eine Stereo-Mikroskopieanordnung nach Item 14, wobei wenigstens eine Einstellvorrichtung zum Abgleich der optischen Weglängen vorgesehen ist.
16. Eine Stereo-Mikroskopieanordnung nach Item 12 bis 15, wobei das Strahlvertauschungssystem aus den Strahlengängen der Okularanordnungen entfernbar ist und statt dessen in die Strahlengänge der Okularanordnungen ein Bildumkehrsystem einführbar ist, welches die Bildorientierung des der linken und der rechten Okularanordnung zugeführten Strahlenbündels jeweils umkehrt.
17. Eine Stereo-Mikroskopieanordnung nach Item 16, wobei das Strahlvertauschungssystem und das Bildumkehrsystem für die den Okularanordnungen zugeführten Strahlenbündel im wesentlichen gleiche Okular-Zwischenbildlagen bereitstellen.
18. Eine Stereo-Mikroskopieanordnung nach Item 12 bis 17, ferner umfassend ein vor der Objektivanordnung anordenbares Vorsatzlinsensystem zur Erzeugung eines seiten- und höhenverkehrten Zwischenbilds eines zu betrachtenden Objekts in der Objektebene der Objektivanordnung.
19. Eine Stereo-Mikroskopieanordnung nach Item 12 bis 18,
wobei das Strahlvertauschungssystem
- einen ersten Spiegel zur Umlenkung eines ersten der beiden den Okularanordnungen zugeführten Strahlenbündels in eine Richtung quer zu dessen Einfallsrichtung in das Strahlvertauschungssystem,
- einen zweiten Spiegel zur Umlenkung dieses umgelenkten Strahlenbündels in eine Richtung parallel zu der Einfallsrichtung,
- einen dritten Spiegel zur Umlenkung dieses umgelenkten Strahlenbündels in eine Richtung entgegengesetzte zu der Richtung nach Umlenkung an dem ersten Spiegel und
- einen vierten Spiegel zur Umlenkung dieses umgelenkten Strahlenbündels in Richtung und koaxial zu dem zweiten der beiden Strahlenbündel,
- einen fünften Spiegel zur Umlenkung des zweiten der beiden Strahlenbündel in eine Richtung quer zu dessen Einfallsrichtung in das Strahlvertauschungssystem,
- einen sechsten Spiegel zur Umlenkung dieses umgelenkten Strahlenbündels in eine Richtung entgegengesetzt zu der Einfallsrichtung,
- einen siebten Spiegel zur Umlenkung dieses umgelenkten Strahlenbündels in eine Richtung entgegengesetzt zu der Richtung nach Umlenkung an dem fünften Spiegel und
- einen achten Spiegel zur Umlenkung dieses umgelenkten Strahlenbündels in Richtung und koaxial zu dem ersten der beiden Strahlenbündel umfaßt.

## Patentansprüche

1. Stereo-Mikroskopieanordnung zur Betrachtung eines Objekts oder eines von einem Objekt erzeugten Zwischenbilds, insbesondere in der Mikrochirurgie, umfassend:
eine Objektivanordnung (909) mit einer Objektebene (905) zur Anordnung des zu betrachtenden Objekts bzw. Zwischenbilds, eine linke Okularanordnung (903), der ein in die Objektivanordnung links eintretendes Strahlenbündel (907) zugeführt wird, und eine rechte Okularanordnung (904), der ein in die Objektivanordnung rechts eintretendes Strahlenbündel (908) zugeführt wird,
**dadurch gekennzeichnet, daß**
in den Strahlengängen der Okularanordnungen ein Strahlvertauschungssystem (1) vorgesehen ist, welches das der linken Okularanordnung zugeführte Strahlenbündel (5) in die rechte Okularanordnung spiegelt und welches das der rechten Okularanordnung zugeführte Strahlenbündel in die linke Okularanordnung spiegelt,
wobei die dem Strahlvertauschungssystem zugeführten Strahlenbündel und aus dem Strahlvertauschungssystem austretende Strahlenbündel jeweils einander gleiche Bildorientierungen aufweisen,
wobei das Strahlvertauschungssystem solche optischen Komponenten ((15,41), (35,61)) aufweist, die bezüglich einer Mittelebene (3) zwischen den Strahlenbündeln auf diese symmetrisch wirken, sowie auch solche optischen Komponenten (25, 51) aufweist, die bezüglich der Mittelebene (3) zwischen den Strahlenbündeln auf diese unsymmetrisch wirken.

2. Stereo-Mikroskopieanordnung nach Anspruch 1, wobei die linke und die rechte Okularanordnung jeweils einen Bereich mit einem konvergenten Strahlengang aufweisen und wobei das Strahlvertauschungssystem in dem konvergenten Strahlengang angeordnet ist.

3. Stereo-Mikroskopieanordnung nach Anspruch 1 oder 2, wobei das Strahlvertauschungssystem für das der rechten Okularanordnung zugeführte Strahlenbündel und für das der linken Okularanordnung zugeführte Strahlenbündel jeweils im wesentlichen gleiche optische Weglängen bereitstellt.

4. Stereo-Mikroskopieanordnung nach Anspruch 3, wobei wenigstens eine Einstellvorrichtung (65) zum Abgleich der optischen Weglängen vorgesehen ist.

5. Stereo-Mikroskopieanordnung nach einem der Ansprüche 1 bis 4, wobei das Strahlvertauschungssystem aus den Strahlengängen der Okularanordnungen entfernbar ist und statt dessen in die Strahlengänge der Okularanordnungen ein Bildumkehrsystem (73) einführbar ist, welches die Bildorientierung des der linken und der rechten Okularanordnung zugeführten Strahlenbündels jeweils umkehrt.

6. Stereo-Mikroskopieanordnung nach Anspruch 5, wobei das Strahlvertauschungssystem und das Bildumkehrsystem für die den Okularanordnungen zugeführten Strahlenbündel im wesentlichen gleiche Okular-Zwischenbildlagen bereitstellen.

7. Stereo-Mikroskopieanordnung nach einem der Ansprüche 1 bis 6, ferner umfassend ein vor der Objektivanordnung anordenbares Vorsatzlinsensystem (115) zur Erzeugung eines seiten- und höhenverkehrten Zwischenbilds eines zu betrachtenden Objekts in der Objektebene der Objektivanordnung.

8. Stereo-Mikroskopieanordnung zur Betrachtung eines Objekts oder eines von einem Objekt erzeugten Zwischenbilds, insbesondere in der Mikrochirurgie, umfassend:
eine Objektivanordnung mit einer Objektebene zur Anordnung des zu betrachtenden Objekts bzw. Zwischenbilds, und ein Strahlvertauschungs- und Bildumkehrsystem (89; 89b), das ein von der Objektebene nach links in Richtung zu der Objektivanordnung gerichtetes Strahlenbündel der Objektivanordnung rechts zuführt, das ein von der Objektebene nach rechts in Richtung zu der Objektivanordnung gerichtetes Strahlenbündel der Objektivanordnung links zuführt, und das Bildorientierungen der beiden Strahlenbündel jeweils umkehrt,
ferner umfassend ein vor der Objektivanordnung anordenbares Vorsatzlinsensystem zur Erzeugung eines seiten- und höhenverkehrten Zwischenbilds eines Augenfundus in der Objektebene der Objektivanordnung, wobei das Strahlvertauschungs- und Bildumkehrsystem (89; 89b) und das Vorsatzlinsensystem aus dem Strahlengang vor der Objektivanordnung entfernbar sind, und wobei geometrische Abmessungen und optische Medien der Strahlvertauschungs- und Bildumkehr-Anordnung (89; 89b) und Brennweiten des Vorsatzlinsensystems (115) derart abgestimmt sind, dass bei in dem Strahlengang angeordnetem Strahlvertauschungs- und Bildumkehrsystem und Vorsatzlinsensystem das Zwischenbild des Augenfundus in der Objektebene der Objektivanordnung anordenbar ist und dabei die Objektivanordnung von dem Auge einen ersten Abstand (b2 + b4) aufweist, und dass bei aus dem Strahlengang entfernten Strahlvertauschungs- und Bildumkehrsystem und Vorsatzlinsensystem die Cornea (121) des Auges in der Objektebene (117) der Objektivanordnung anordenbar ist und dabei die Objektivanordnung von dem Auge einen zweiten Abstand (b1) aufweist, und dass der erste und der zweite Abstand im wesentlichen gleich sind.

9. Stereo-Mikroskopieanordnung zur Betrachtung eines Objekts oder eines von einem Objekt erzeugten Zwischenbilds, insbesondere in der Mikrochirurgie, umfassend:
eine Objektivanordnung mit einer Objektebene zur Anordnung des zu betrachtenden Objekts bzw. Zwischenbilds, und ein Strahlvertauschungs- und Bildumkehrsystem (89; 89b), das ein von der Objektebene nach links in Richtung zu der Objektivanordnung gerichtetes Strahlenbündel der Objektivanordnung rechts zuführt, das ein von der Objektebene nach rechts in Richtung zu der Objektivanordnung gerichtetes Strahlenbündel der Objektivanordnung links zuführt, und das Bildorientierungen der beiden Strahlenbündel jeweils umkehrt,
wobei das Strahlvertauschungs- und Bildumkehrsystem aus dem Strahlengang vor der Objektivanordnung entfernbar ist, wobei bei in dem Strahlengang angeordnetem Strahlvertauschungs- und Bildumkehrsystem die Objektebene der Objektivanordnung von der Objektivanordnung einen dritten Abstand (b2) aufweist, wobei bei aus dem Strahlengang entferntem Strahlvertauschungs- und Bildumkehrsystem die Objektebene von der Objektivanordnung einen vierten Abstand (b1) aufweist, und wobei eine Differenz zwischen dem dritten (b2) und dem vierten (b1) Abstand einen vorbestimmten Wert aufweist.

10. Stereo-Mikroskopieanordnung zur Betrachtung eines Objekts oder eines von einem Objekt erzeugten Zwischenbilds, insbesondere in der Mikrochirurgie, umfassend:
eine Objektivanordnung mit einer Objektebene zur Anordnung des zu betrachtenden Objekts bzw. Zwischenbilds, eine linke und eine rechte Okularanordnung, und ein Strahlvertauschungs- und Bildumkehrsystem, das ein in die Objektivanordnung links eintretendes Strahlenbündel der rechten Okularanordnung zuführt, das ein in die Objektivanordnung rechts eintretendes Strahlenbündel der linken Okularanordnung zuführt und das Bildorientierungen der beiden Strahlenbündel jeweils umkehrt,
ferner umfassend ein zwischen der Objektivanordnung (909) und den beiden Okularanordnungen angeordnetes Vergrößerungsänderungssystem (911), wobei das Strahlvertauschungs- und Bildumkehrsystem zwischen der Objektivanordnung und dem Vergrößerungsänderungssystem angeordnet ist,
optional ferner umfassend ein vor der Objektivanordnung anordenbares Vorsatzlinsensystem (115) zur Erzeugung eines seiten- und höhenverkehrten Zwischenbilds des zu betrachtenden Objekts in der Objektebene der Objektivanordnung.
